# EUROPÄISCHE PATENTSCHRIFT

(11) **EP 1 479 363 B1**
(45) Veröffentlichungstag und Bekanntmachung des Hinweises auf die Patenterteilung: **17.05.2006**
(21) Anmeldenummer: 03405697.8
(22) Anmeldetag: 25.09.2003
(51) Int. Cl.: A61H 1/00, A61F 13/15

(54) **Einbringen von Kosmetika in die Haut**
Application of cosmetics in the skin
Application de cosmétiques dans la peau

(43) Veröffentlichungstag der Anmeldung: 24.11.2004
(73) Patentinhaber: Power-Wrap International Trading GmbH, 1060 Vienna (AT)
(72) Erfinder: Millet, Oswald, 1060 Wien (AT)
(74) Vertreter: Frei Patent Attorneys

(56) Entgegenhaltungen:
- WO-A-02/053084
- WO-A-03/028599
- DE-U- 20 115 485
- GB-A- 979 184
- US-A- 6 080 127

## Beschreibung

Die Erfindung betrifft ein Verfahren zum Eintreiben von feuchtigkeitspendenden und/oder kosmetischen Wirkstoffen in die Haut, die Verwendung einer Kompressionsbinde oder Kompressionsbandage in einem solchen Verfahren, sowie eine Vorrichtung und eine Einrichtung zum Eintreiben von feuchtigkeitsspendenen und/oder kosmetischen Wirkstoffen in die Haut.

Es ist bekannt, die Haut straffende und glättende Cremen, Gels, Lotionen oder Öle auf den Körper aufzubringen. Damit erzielte kosmetische Effekte können eine Glättung der Haut, eine Entschlackung des Gewebes und ein Bekämpfen von Besenreisern einschliessen.

Nachteilig ist dabei, dass das Einziehen grösserer Mengen von kosmetischen Cremen, Gels, Lotionen oder Öle in die Haut ein langsamer Prozess ist und die Wirkung entsprechend langsam und schwach eintritt. Ausserdem beinhalten solche Produkte oft beispielsweise starke Reizstoffe, die als Schleuse für die Wirkstoffe eingesetzt werden und eine intensive Durchblutung von Haut und Unterhautbindegewebe zu erzielen. Starke Reizstoffe können sich aber unangenehm anfühlen.

Aus der US-Patentschrift 6,080,127 sind Verfahren und Vorrichtungen bekannt, bei denen mittels Vibrationen das einziehen von Substanzen in Haarfollikel begünstigt wird.

Die internationale Offenlegungsschrift WO 03/028599 offenbart eine Kompressionsmanschette, deren Material als Trägermatrix für eine mikroverkapselte chemische, kosmetische oder biologische Substanz dienen kann.

Die Schriften WO 02/053084 und DE 2001 15 485 zeigen Vorrichtungen für die vibratorische Stimulation des menschlichen Körpers.

Der Erfindung liegt der Gedanke zu Grunde, die Feuchtigkeit spendenden Stoffe und/oder kosmetischen Wirkstoffe nicht passiv einziehen zu lassen, sondern aktiv einzubringen, also einzutreiben.

Erfindungsgemäß wird dies durch ein Verfahren mit den Merkmalen des Anspruchs 1 und durch eine Einrichtung mit den Merkmalen des Anspruchs 4 gelöst. Vorteilhafte Ausgestaltungen sind in den Unteransprüchen spezifiziert.

Die Erfindung stellt daher ein Verfahren zum Einbringen kosmetischen Wirkstoffen sowie eine Einrichtung zum Einbringen von Feuchtigkeit spendenden Substanzen und kosmetischen Wirkstoffen zur Verfügung, welches Nachteile bestehender Vorgehensweisen überwindet und welches insbesondere auf grossen Flächen effizient wirkt.

Dies wird dadurch bewirkt, dass die feuchtigkeitsspendenden Substanzen und/oder kosmetischen Wirkstoffe die Haut eines menschlichen Körpers eingetrieben werden, indem die Haut, die mit diesen Substanzen in Kontakt ist, an eine Kompressionswand angelegt wird, und indem ein mit der Haut verbundenes Gewebe anschliessend in Schwingungen versetzt wird, wobei die Kompressionswand so anliegen kann, dass ein Ausschwingen (im Sinne von auslenkendes Schwingen) des Gewebes verhindert wird.

Die Schwingungen werden aufgrund der Kompressionswand zurück ins Gewebe reflektiert. Das Gewebe wird mindestens im Takt der Anregungsfrequenz gegen die Kompressionswand gedrückt, wodurch einzubringende Substanzen eingetrieben (fast schon 'eingeklopft') werden.

Der Begriff 'kosmetische Wirkstoffe' ist hier breit aufzufassen. Er umfasst insbesondere jegliche die Hautoberfläche kosmetisch wirksam beeinflussende oder die Haut straffende Substanzen, entschlackende Substanzen aber auch rückfettende Substanzen etc.

Mit 'Kompressionswand' ist ein häufig hüllenartiges, also flaches, biegbares und mindestens teilweise umhüllendes, ans Gewebe eng anlegbares Element gemeint. Häufig wird die Kompressionswand eine gewisse Elastizität aufweisen, damit sie straff gespannt werden kann.

Dieses Vorgehen hat grundlegende Vorteile:
- Indem Schwingungen im Gewebe angeregt werden, kann die Wirkung grossflächig sein, ohne dass ein grosser technischer Aufwand betrieben werden müsste. Insbesondere müssen nicht grossflächig oder gar auf der ganzen Oberfläche des Gewebes Mittel zum Anregen der Schwingungen vorhanden sein. Es genügt, die Kompressionswand an grosse Oberflächen des Gewebes anliegen zu lassen. Dies kann bspw. durch grossflächige, straffe elastische oder nicht elastische Binden oder Bandagen bewerkstelligt werden. Diese Binden oder Bandagen können ausserdem direkt mit den feuchtigkeitspendenden Substanzen und/oder Wirkstoffen getränkt oder belegt sein.
- Durch das aktive Eintreiben der feuchtigkeitspendenden Substanzen und/oder kosmetischen Wirkstoffe können grössere Mengen in die Haut eingearbeitet werden.
- Es lässt sich zeigen, dass der menschliche Körper, wenn er mindestens in Teilbereichen zu Schwingungen angeregt wird, automatisch trainiert wird. Bspw. beim Stehen auf einer dreidimensional schwingenden Unterlage wird der Körper Bewegungen der Unterlage reflexartig ausgleichen. Der dabei erzielbare Trainingseffekt ist beachtlich. Ähnliches gilt für Elektrostimulation. Das erfindungsgemässe Verfahren ist also fast ohne Zusatzaufwand kombinierbar mit einer Trainingsmethode, was die kosmetische Wirkung natürlich noch verstärkt (Fettabbau und lokale Muskelstraffung).
- Der erfindungsgemässe Ansatz erlaubt ohne Weiteres ein Anpassen an die Konsistenz des Gewebes, in welches die feuchtigkeitspendenden Substanzen und/oder kosmetischen Wirkstoffe eingetrieben werden sollen. Es ist bekannt, dass je nach Spannung des Gewebes sehr unterschiedliche Schwingungen angeregt werden können. Beispielsweise sind bei sehr schlaffem Gewebe niederfrequente Schwingungen möglich (Beispiel: "Schwabbelbauch"). Durch Anpassen der Anregungsfrequenz kann das erfindungsgemässe Verfahren also bei Bedarf auf die entsprechende Person angepasst werden.

Die Erfindung bedient sich gemäss einer Ausführungsform der Tatsache, dass bei 'zufälligen' Schwingungsanregungen eine Vielzahl von Schwingungen ausgelöst wird. Die Schwingungen sind u.a. sowohl als stehende als auch als fortlaufende Wellen im Gewebe des Körpers, die fortlaufenden Wellen haben unterschiedliche Wellenausbreitungsrichtungen. Unter den angeregten Wellen gibt es transversale, entlang der Oberfläche ausgebildete Wellen, deren Wellenlänge bspw. kürzer ist als eine Abmessung der durch die Kompressionswand abgedeckte Fläche. Solche Wellen werden durch die Kompressionswand behindert; die Kompressionswand verhindert Auslenkungen. An denjenigen Stellen, an welchen bei einem freien Schwingen eine Auslenkung bewirkt würde, wird das Gewebe gegen die Kompressionswand gedrückt und ggf. leicht komprimiert. Dies bewirkt ein Eintreiben der einzubringenden Substanzen ins Gewebe.

Die Anregung von Schwingungen kann dadurch geschehen, dass die Person auf eine Unterlage steht (oder sitzt oder liegt), die Schwingungen ausführt. Es hat sich gezeigt, dass das Verfahren besonders gut wirkt, wenn die Unterlage Schwingungen in mehreren Richtungen ausführt. Bspw. kann ein Produkt verwendet werden, bei denen eine Platte dreidimensional vibriert. Als günstig haben sich Schwingungen mit Frequenzen zwischen 10 Hz und 150 Hz, beispielsweise zwischen 20 Hz und 90 Hz, speziell zwischen 30 Hz und 50 Hz herausgestellt. Ein besonders vorteilhafter Effekt wird erzielt, wenn sich die Vibrationen nicht auf eine einzige regelmässige Schwingung beschränken, sondern in mehreren Dimensionen und möglicherweise nicht streng periodisch verlaufen. Sowohl Frequenz als auch Amplitude können auf die Bedürfnisse der Person abgestimmt werden, beispielsweise auf die Straffheit des Gewebes.

Verfahren zum Anregen von Vibrationen in einer Unterlage, beispielsweise einer Platte sind bekannt. Sie können zum Beispiel auf drehenden exzentrischen Abstandhaltern zwischen der vibrierenden Platte und einer ortsfesten Basis beruhen. Die Details von solchen Verfahren sollen hier nicht näher erläutert werden.

Ergänzend zu dieser Art der Schwingungsanregung kann auch elektrostimuliert werden. Durch Elektrostimulation wird eine Muskelkontraktionstätigkeit angeregt. Auch hier kann die Frequenz so gewählt werden dass die gewünschten anzuregenden Schwingungen besonders intensiv - fast resonant - angeregt werden und/oder dass ein besonderer Trainingseffekt erzielbar ist.

Schliesslich gibt es noch die Möglichkeit der Anregung durch ein Bandvibrationsgerät. Ein solches kann auf oder mindestens teilweise neben dem Kompressionswickel/der Kompressionsbandage angelegt werden.

Im Folgenden werden Ausführungsbeispiele der Erfindung anhand von allesamt sehr schematischen Zeichnungen näher erläutert. In den Zeichnungen zeigen:
- Fig. 1 eine Person mit verschiedenartigen Kompressionswickeln
- Fig. 2 eine Person mit mehreren Kompressionsbandagen
- Fig. 3 eine Person auf einer vibrierenden Unterlage
- Fig. 4 das Prinzip der Verhinderns von auslenkenden Schwingungen
- Fig. 5 eine Person mit mehreren Kompressionswickeln oder -bandagen und mit Mitteln zur Elektrostimulation.

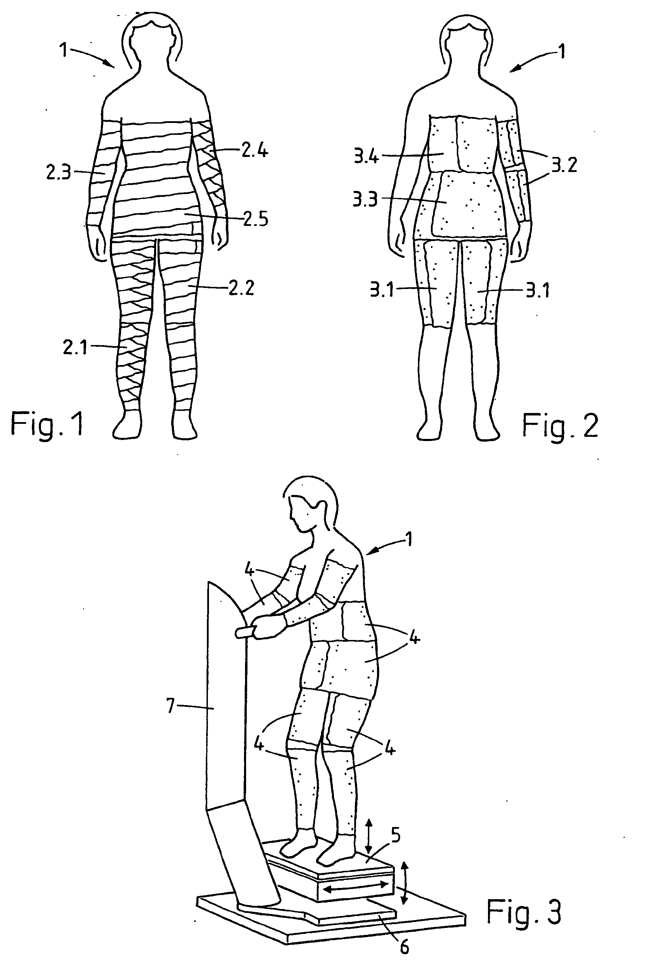

Die in **Figur 1** dargestellte Person 1 ist mit straff anliegenden Kompressionsbinden 2.1, 2.2, 2.3, 2.4, 2.5 versehen. Diese können unter Benutzung von an sich bekannten, aber einen verhältnismässig grossen Elastizitätsmodul aufweisenden und daher straff spannenden elastischen Binden oder unter Benutzung anderer mindestens wenig elastischer Materialien gefertigt sein. Unter den Kompressionswickeln sind auf die Haut der Person 1 feuchtigkeitsspendende und/oder kosmetisch wirksame Substanzen, eventuell in einem geeigneten Lösungsmittel, aufgebracht worden; alternativ oder ergänzend dazu kann auch das Material der Binden mit Substanzen dieser Art getränkt sein. Es ist auch möglich, mehrere Produkte nacheinander auf die Haut aufzutragen.

Über den Wickel wird ggf. (nicht dargestellt) eine äussere Hülle angebracht, welche bspw. folienartig ausgebildet sein kann. Diese Hülle kann ebenfalls straff gespannt sein und die Wirkung der Kompressionswickel noch verstärken. Ausserdem kann sie im Wesentlichen flüssigkeitsdicht sein und dadurch verhindern, dass eventuell über den Wickel getragene Kleidungsstücke durch Stoffe, mit denen der Wickel getränkt ist oder sich vollsaugt, verschmutzt werden.

In **Figur 2** trägt die Person anstelle von Kompressionsbinden Kompressionsbandagen 3.1, 3.2, 3.3, 3.4. Diese sind ebenfalls so ausgebildet, dass sie straff an der Haut der Person anliegen können. Sie können bspw. ähnlich wie Blutdruckmanschetten einen reversibel betätigbaren Verschluss, bspw. einen sog. 'Klett'-Verschlüsse o.ä. aufweisen. Auch ein solcher Verschluss kann bei mindestens leicht elastischem Bandagenmaterial und wenn mit etwas Kraft angelegt wie erforderlich ermöglichen, dass die Bandage satt am Gewebe anliegt. Die Bandagen können auch zusätzliche Mittel aufweisen, mit welchen das satte Anliegen/der Druck auf das Gewebe noch verbessert bzw. verstärkt werden. Solche zusätzlichen Mittel können sich selbst aufblasende oder aufzublasende Druckkörper im Innern der Bandage etc. beinhalten.

In der Zeichnung dargestellt sind Bandagen, die manschettenartig und verschliessbar sind. Es sind aber auch geschlossene oder gar strumpfartige Bandagen denkbar, die, ggf. mittels geeigneter Hilfsmittel, übergestreift werden können, ähnlich wie Stützstrümpfe. Die Bandagen können eine äussere, im Wesentlichen flüssigkeitsdichte und strapazierfähige Schicht aufweisen, welche eine separate äussere Hülle überflüssig macht. Sie können aber auch ähnlich wie die Wickel durch eine separate äussere Hülle ergänzt werden.

Wenn es nicht notwendig ist, dass über die Wickel/Bandagen ein Kleidungsstück getragen wird, kann in beiden Fällen auf die Hülle/die äussere Schicht verzichtet werden.

In **Figur 3** steht die Person 1 mit Wickeln 4 versehen auf einer dreidimensional vibrierenden Unterlage, nämlich einer Platte 5. Die Platte ist auf einer ortsfesten Basis 6 angebracht. Zwischen der Platte 5 und der Basis 6 befinden sich Antriebsmittel, durch welche die Platte in Schwingungen in verschiedenen Auslenkungsrichtungen Ausprägungen versetzt wird. Durch Vibrationen der Platte 5 werden Schwingungen im Körper der Person angeregt. Die Person hält sich an einer optionalen Stützsäule 7 fest, damit sie sich nicht darauf konzentrieren muss, das Gleichgewicht zu bewahren.

Die Vielzahl Schwingungen, welche in einem inhomogenen Gewebe mit - mathematisch gesehen - irregulärer Form angeregt wird, lässt sich nicht einfach beschreiben. Ganz allgemein kann aber ein Schwingungszustand durch eine lineare (d.h. mathematisch unabhängige) Überlagerung von sog. Eigenschwingungen angenähert werden. Es kann daher illustrativ sein, einzelne Schwingungen aus der simultan angeregten Vielzahl von Schwingungen für sich zu betrachten. Zur Illustration werden in der Figur Schwingungen dargestellt, die transversalen, entlang der Gewebeoberfläche verlaufenden Wellen entsprechen. Für solche Wellen gilt, dass die Fortbewegungsgeschwindigkeit und damit bei gegebener Anregungsfrequenz auch der Abstand zwischen zwei "Wellenbergen" proportional zur Wurzel der Spannung des Gewebes (als Kraft) ist. Das heisst, je schlaffer das Gewebe, desto langsamer die Ausbreitung der Wellen und desto weiter auseinander die 'Wellenberge'.

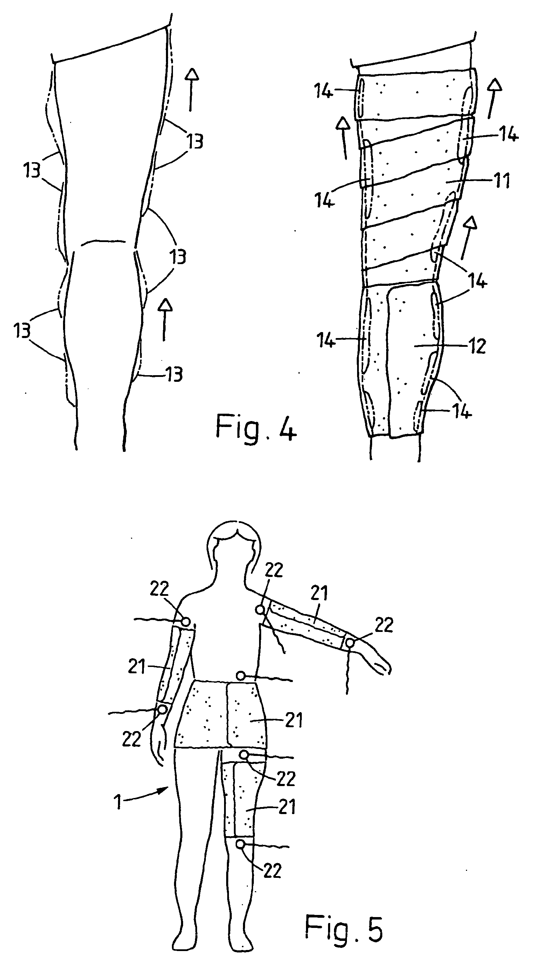

Die Wirkung der Kompressionswand bei Schwingungsanregungen wird in Figur 4 sehr schematisch dargestellt. Beide Zeichnungen der Figur zeigen zur Illustration das Bein einer auf einer vibrierenden Unterlage stehenden Person von hinten. In der rechten Zeichnung trägt die Person je einen Schwingungen behindernden Wickel 11 und eine Manschette 12, während in der rechten Zeichnung das Gewebe frei ausschwingen kann. Das freie, auslenkende Schwingen ist durch sich in Pfeilrichtung fortbewegende Wellenauslenkungen 13 dargestellt.

Im der rechten Zeichnung kann das Gewebe nicht auslenkend schwingen, aufgrund der vom Kompressionswickel bzw. der Kompressionsbandage gebildeten Kompressionswand. Wenn am Gewebe eine solche Kompressionswand anliegt wirkt sich diese auf solche Schwingungen wie folgt aus: Durch die Kompressionswand wird ein Schwingen des Gewebes mit Auslenkung (hier auch 'Ausschwingen" genannt, was aber nicht gleichzusetzen ist mit ,abklingendem Schwingen') verhindert. Statt dessen wird das Gewebe ungefähr an den Orten, wo Auslenkungen vorhanden wären, wenn keine Kompressionswand vorhanden wäre, lokal gegen die Kompressionswand gedrückt und dabei zusammengedrückt und je nach Kompressibilität leicht komprimiert. Dies hat eine die einzubringenden Substanzen eintreibende Wirkung auf die Haut. Komprimierte Stellen 14 im Gewebe sind in der Figur schematisch dargestellt.

Natürlich haben Kompressionswände auch einen Einfluss auf Ausbildung und Ausbreitungseigenschaften der angeregten Schwingungen. Auf diesen Umstand wird hier zur Vereinfachung der lediglich zur Illustration dienenden Diskussion anhand von Figur 4 nicht eingegangen.

Es ist nicht notwendig, dass Schwingungen vorhanden sein müssen, die propagierende Auslenkungen mit sich bringen, falls keine Kompressionswand vorhanden ist. Ebensogut können sie als ,stehende Wellen' ausgebildet sein, ähnlich der Schwingungen, die sich an einem beidseitig eingespannten Seil ergeben etc. Wie erwähnt lässt sich der tatsächliche Schwingungszustand ohnehin als Überlagerung von einer Vielzahl von möglichen Schwingungszuständen sehen.

In **Figur 5** ist eine Person dargestellt, welche sehr schematisch gezeichnete Kompressionswickel 21 sowie ebenfalls sehr schematisch gezeichnete Elektroden 22 eines Elektrostimulationsgeräts trägt. Das Elektrostimulationsgerät tritt an die Stelle der vibrationsfähigen Platte gemäss Fig. 3 und dient ebenfalls zum Anregen von Schwingungen im Gewebe. Das Anregen von Muskelkontrationen über Elektrostimulation ist an sich bekannt und wird hier nicht weiter erläutert.

Anstelle der vibrationsfähigen Platte und des Elektrostimulationsgerätes sind noch weitere Mittel zum Anregen von Schwingungen in einem Gewebe denkbar. Wichtig im Sinne der Erfindung ist lediglich, dass das Gewebe selbst zu Schwingungen angeregt wird, und zwar im Allgemeinen so, das es auf der ganzen Fläche der Kompressionswickel bzw. -bandagen schwingt und nicht nur punktuell.

Eine Einrichtung zum Eintreiben von feuchtigkeitsspendenden Substanzen in die Haut weist folgende Bestandteile auf:
- Ein Mittel zum Anregen von Schwingungen in einem menschlichen Gewebe, bspw. eine vibrationsfähige Platte oder ein Elektrostimulationsgerät.
- Mindestens eine kompressionsfähige Binde und/oder Bandage. Eine kompressionsfähige Bandage kann mit einer äusseren im Wesentlichen flüssigkeitsdichten Hülle versehen sein.
- Vorzugsweise feuchtigkeitsspendende und/oder kosmetisch wirksame Substanzen. Diese sind entweder direkt in den Binden/Bandagen vorhanden (d.h. letztere sind mindestens teilweise mit ihnen getränkt) oder sie werden in separaten Behältnissen geliefert.
- Eventuell zusätzliches Hüllmaterial, im Wesentlichen flüssigkeitsdicht, beispielsweise eine Folie.

Die feuchtigkeitsspendenden und/oder kosmetisch wirksamen Substanzen können als Gele, Emulsionen Cremen, Öle, Lotionen etc. vorliegen und eine breite Kombination von Inhaltsstoffen aufweisen. Mögliche gewebe- und hautpflegende Inhaltsstoffe sind aus der Kosmetik bekannt. Sie können Wasser, Fette und/oder Alkohole und mindestens einige aus der Gruppe der Extrakte aus Ginkoblättern und Ginsengwurzeln, Macadamia, Malva sylvestris, Minze, Melisse, Ringelblume, Algen, Oliven, Avocado, Rosskastanie, Blasentang, Brennessel, Schachtelhalm, Jojobaöl, Maisöl, Mandelöl, Weizenkeimöl,
weiterer natürliche Ingredienzen
sowie synthetischer Wirkstoffe
beinhalten.

## Patentansprüche

1. Verfahren zum Einbringen von kosmetischen Wirkstoffen in die Haut eines menschlichen Körpers, in welchem die Haut mit den Substanzen und/oder Wirkstoffen in Kontakt gebracht, eine Kompressionsbinde (2.1-2.5) oder Kompressionsbandage (3.1-3.4) angelegt wird, wodurch eine Kompressionswand gebildet wird, **dadurch gekennzeichnet, dass** ein mit der Haut verbundenes Gewebe dadurch in Schwingungen versetzt wird, dass der menschliche Körper auf einer schwingenden Unterlage (5) plaziert wird.

2. Verfahren nach Anspruch 1, **dadurch gekennzeichnet, dass** um die elastische Binde oder Bandage herum eine Folie angebracht wird.

3. Verfahren nach Anspruch 1 oder 2, **dadurch gekennzeichnet, dass** das Gewebe zusätzlich zu Schwingungen angeregt wird, indem eine Kontraktionstätigkeit von Muskeln durch Elektrostimulation ausgelöst wird.

4. Einrichtung zum Einbringen von feuchtigkeitsspendenden Substanzen und/oder von kosmetischen Wirkstoffen in Gewebe von menschlichen Körpern, umfassend mindestens eine Kompressionsbinde und/oder Kompressionsbandage (2.1-2.5, 3.1-3.4, 4) **gekennzeichnet durch** ein Schwingungsanregungsgerät mit Mitteln zum in-Schwingung-Versetzen von menschlichem Gewebe, welches Schwingungsgerät eine Unterlage (5) mit elektrisch betreibbaren Antriebsmitteln umfasst, welche Antriebsmittel im Betrieb die Unterlage zu Schwingungen anregt, wobei Antriebsmittel und Unterlage so ausgebildet sind, dass ein menschlicher Körper darauf plaziert werden kann.

5. Einrichtung nach Anspruch 4, **dadurch gekennzeichnet, dass** ein Mensch auf der Unterlage stehen kann, wenn das Schwingungsanregungsgerät in Betrieb ist.

## Claims

1. Method for driving cosmetic active ingredients into the skin of a human body, in which the skin is brought into contact with the substances and/or active ingredients, a compression wrap (2.1-2.5) or compression bandage (3.1-3.4), through which a compression element is formed, **characterized in that** a tissue connected to the skin is set into oscillation by placing the human body on a oscillating support (5).

2. Method according to claim 1, **characterized in that** a film is applied around the compress or elastic bandage.

3. Method according to claims1 or 2, **characterized in that** the tissue is additionally excited into oscillation, **in that** a contraction activity of muscles is triggered by electro-stimulation.

4. Means for driving moisturising substances and/or cosmetic active ingredients into tissue of human bodies, comprising at least one compression wrap and/or compression bandage (2.1-2.5, 3.1-3.4, 4) **characterized by** an oscillation excitation apparatus with means for setting human tissue into oscillation, which oscillation excitation apparatus comprises a support (5) with electrically driven driving means, which driving means, when in operation cause the support to oscillate, wherein driving means and support are designed such that a human body may be placed on top.

5. Means according to claim 4, **characterized in that** a human being may stand on the support when the oscillation excitation apparatus is in operation.

## Revendications

1. Procédé d'introduction de principes actifs cosmétiques dans la peau d'un corps humain, dans lequel on met la peau en contact avec les substances et/ou les principes actifs, on pose une bande de compression (2.1-2.5) ou un bandage de compression (3.1-3.4), ce qui a pour effet de former une paroi de compression, **caractérisé en ce qu'**on imprime des vibrations à un tissu relié à la peau en plaçant le corps humain sur un support vibrant (5).

2. Procédé selon la revendication 1, **caractérisé en ce qu'**on applique un film autour de la bande ou du bandage élastique.

3. Procédé selon la revendication 1 ou 2, **caractérisé en ce que** le tissu est amené à vibrer en supplément par déclenchement d'une action de contraction de muscles par électrostimulation.

4. Dispositif d'introduction de substances hydratantes et/ou de principes actifs cosmétiques dans le tissu de corps humains, comprenant au moins une bande de compression et/ou un bandage de compression (2.1-2.5, 3.1-3.4, 4), **caractérisé par** un appareil d'activation de vibrations avec des moyens pour imprimer des vibrations à un tissu humain, lequel appareil vibrant comprend un support (5) avec des moyens d'entraînement à actionnement électrique, lesquels moyens d'entraînement activent le support en vibrations en cours de service, les moyens d'entraînement et le support étant conçus de façon à pouvoir placer dessus un corps humain.

5. Dispositif selon la revendication 4, **caractérisé en ce qu'**un humain peut se mettre debout sur le support, lorsque l'appareil d'activation de vibrations est en service.
